# FASCICULE DE BREVET EUROPEEN

(11) **EP 2 765 154 B1**
(45) Date de publication et mention de la délivrance du brevet: **03.10.2018**
(21) Numéro de dépôt: 14164589.5
(22) Date de dépôt: 11.12.2008
(51) Int. Cl.: C08J 3/22, C08K 3/18, C08K 3/30, C08K 3/32, C08K 3/34, C08K 5/54, C08K 5/56, D01F 1/10, D01F 6/60, A61F 13/06, A61K 8/23, A61K 8/29, A61K 8/88, A61Q 19/06, A61K 8/26, A61K 8/02, A61K 9/00, A61K 31/785

(54) **Utilisation d'un article à base d'une composition polymérique**
Verwendung eines Artikels basierend auf einer Polymerzusammensetzung
Use of an article based on a polymeric composition

(30) Priorité: 14.12.2007 FR 0708724; 30.07.2008 FR 0804334
(43) Date de publication de la demande: 13.08.2014
(62) Demande divisionnaire de: 11173204.6
(73) Titulaire: Rhodia Poliamida E Especialidades Ltda, Sao Paulo - SP (BR)
(72) Inventeur: Canova, Thomas, SAO PAULO (BR); Bizaroli De Mendonca, Dany, SAO PAULO (BR); Cordeiro Bastos, Tarcis, SAO PAULO (BR)
(74) Mandataire: Casalonga

(56) Documents cités:
- EP-A- 1 291 405
- WO-A-2007/055432
- GB-A- 2 303 375
- JP-A- H10 219 514
- US-A1- 2003 069 618
- US-B1- 6 316 102

## Description

La présente invention se rapporte à une composition polymérique qui comprend l'utilisation d'additifs ayant des propriétés d'émission et/ou absorption de radiation dans la région de l'infrarouge long, ainsi qu'à des articles fabriqués à partir de cette composition.

Plus spécifiquement, la présente invention se rapporte à une composition polymérique qui comprend des additifs ayant des propriétés d'émission de radiation dans la région infrarouge, dans une plage de longueur d'onde située entre 2µm et 20µm, ainsi qu'à des articles fabriqués à partir de cette composition. La présente invention se rapporte également à des procédés de fabrication de fils et compositions de polyamide contenant ces additifs, ainsi qu'à des articles tels que des articles textiles comme des tissus ou des tricots fabriqués à partir de ces fils, et l'utilisation de ces articles.

L'interaction entre la radiation dans la région infrarouge, de longueur d'onde entre 2µm et 20µm, et les tissus biologiques attire l'attention des scientifiques depuis deux décennies. D'après les études publiées, la radiation infrarouge dans cette plage entraîne la biostimulation telle que l'augmentation de la microcirculation du sang, la diminution de spasmes musculaires, l'augmentation du métabolisme cellulaire, entre autres. Selon l'un des mécanismes proposés, les cellules des tissus biologiques sont stimulées par un processus de résonance avec la radiation, entraînant une augmentation de la circulation sanguine et une diminution de la concentration d'acide lactique dans les muscles squelettiques humains (Niwa et al 1993: Niwa, Y.; Iizawa O.; Ishimoto K.; Jiang. X.; Kanoh, T.; Electromagnetic Wave Emitting products and "Kikoh" Potentiate Human Leukocyte Functions; International Journal of Biometeorology n° 37, p.133-138, 1993; Perez et Martinez 1995: Pérez, A. C. N., Martinez, A. J. A., Fibra de Photon Plantino. Saint Jean de Compostelle, 1995, p.7-71). D'autres effets tels que l'augmentation du flux sanguin périphérique, l'augmentation de la température du corps ont également été décrits dans la littérature.

Au cours des dernières années, des brevets ont été publiés, revendiquant l'utilisation de matériaux émetteurs de radiation infrarouge dans la plage considérée ci-dessus, pour une application textile. En général, l'application est dirigée vers les effets d'absorption thermique (US5053275), antimicrobiens (US6316102) et concerne l'utilisation de particules de titane métallique (US7201945) ou d'une composition de charges minérales d'oxydes, carbures, sulfates et silicates. Les matériaux cités dans les brevets sont appliqués au moyen d'une solution aqueuse (dans le cas du titane métallique) ou alors mélangés et traités avec un type de résine polymérique et déposés en enduction (« coatings ») (EP1792724) sur des surfaces textiles. Ces applications superficielles ne présentent pas de bonne résistance à l'usage et au lavage, ni de toucher agréable au contact de la peau, notamment sous la forme d'enduction (« coatings ») de résines. Certains brevets cherchent à résoudre ce problème en incorporant les matériaux dans le substrat polymérique et en produisant des filaments au moyen de procédés d'extrusion, étirage et texturation (US4999243, US5880044, WO2007/055432). Cependant, l'utilisation de taux élevés d'oxydes et de carbures de dureté élevée n'est pas adéquate pour la production de fils à partir de composants thermoplastiques, car ils occasionnent une rapide détérioration des organes des machines. La solution trouvée présente également des inconvénients: la coloration de certains carbures et la faible efficacité du traitement (fréquentes ruptures de filaments) compromettent les propriétés mécaniques du fil.

Une alternative proposée par le brevet EP1094136 concerne l'utilisation d'une composition de particules conductrices blanches, d'oxydes blancs émetteurs d'infrarouge et de résines thermoplastiques pour la production de filaments ayant des taux d'oxydes plus bas. Cependant, la composition présente des oxydes de dureté très élevée (au-dessus de 8,0Mohs) et l'utilisation de titanate de potassium, qui se présente en général sous forme d'une poudre fibreuse, pouvant être classée comme fibre respirable, rend la manipulation difficile et désavantageuse du point de vue de l'hygiène et de la santé.

La demande de brevet GB 2 303 375 décrit la préparation de fibres de polyester émettant des radiations dans l'infrarouge lointain, par incorporation dans le polyester d'un mélange d'au moins deux types de particules céramiques qui réfléchissent les rayonnements infrarouge dans la gamme de longueur d'ondes allant de 4 à 25 µm.Les particules sont choisies en particulier parmi ZrO₂, ZrSiO₄, SiO₂ et TiO₂.

Ces fibres procurent, sous l'effet du rayonnement solaire, un effet d'accumulation de chaleur, et un effet de rétention de la chaleur. Ce document ne suggère nullement l'utilisation de telles fibres afin d'augmenter l'élasticité de la peau.

Le document EP 1 291 405 décrit une composition irradiant dans l'infrarouge lointain, et qui présente des propriétés antistatiques améliorées, et qui contient de l'alumine, un composé choisi parmi la silice et le dioxyde de titane, un élément ou composé du platine, du palladium, de l'iridium ou du rhodium, et de l'argent ou un composé de l'argent.

Elle peut être en particulier incorporée dans un polymère synthétique, pour former des fibres qui peuvent être entre autres utilisées pour fabriquer des articles textiles, destinés à avoir un effet chauffant sur le corps, et à faciliter la circulation sanguine. Ce document ne porte nullement sur le problème d'augmenter l'élasticité de la peau.

Par ailleurs, on est toujours à la recherche de produits cosmétiques qui stimulent la peau, en particulier pour des personnes ayant de la « cellulite » (lipodystrophie gynoïde). En effet la « cellulite » est généralement liée à la présence de corps gras dans les cellules grasses présentes sous la peau, qui entraîne une distorsion des tissus sous la peau, et provoque le fameux effet « peau d'orange ». On cherche donc toujours des solutions pour diminuer la cellulite. Ceci afin d'améliorer le confort et le bien-être de la personne.

A cet effet il a été trouvé dans le cadre de la présente invention que des additifs ayant des propriétés d'émission et/ou d'absorption dans la région de l'infrarouge interagissent également avec un autre tissu biologique qui est la peau. En effet ces additifs permettent notamment de diminuer voire de supprimer la cellulite, ce qui est particulièrement intéressant.

La présente invention se rapporte à la production d'une composition polymérique dans laquelle les caractéristiques des additifs absorbeurs et/ou émetteurs d'infrarouge (dans la plage de longueur d'onde située entre 2µm et 20µm) et les taux utilisés résolvent les problèmes posés plus haut concernant la difficulté de traitement des charges minérales et des fils, permettant la production de fils et d'articles textiles offrant confort, bien-être, amélioration de la microcirculation, meilleure homogénéité thermique et diminution de la fatigue musculaire.

Le principal objectif de la présente invention est d'obtenir des fils, fibres, filaments et articles ayant des propriétés de stimulation de la microcirculation sanguine pour offrir une meilleure homogénéité thermique et une diminution de la fatigue musculaire, ainsi qu'une meilleure élasticité de la peau, grâce à l'introduction, dans une matrice polymérique, d'additifs ayant une propriété d'émission et/ou absorption d'infrarouge, de manipulation facile et aisée d'un point de vue industriel. Ces articles permettent notamment de diminuer la cellulite.

La présente invention se rapporte à l'emploi de charges minérales et/ou additifs organiques introduits dans des polymères pour conférer des propriétés d'émission d'infrarouge capables d'offrir une meilleure élasticité de la peau, afin d'apporter confort et bien-être à la personne ; ainsi qu'au procédé pour l'obtention en particulier des fibres, fils et articles obtenus à partir de ces compositions polymériques.

La présente invention a donc pour objet l'utilisation non thérapeutique d'un article à base d'une composition polymérique comprenant un polymère et au moins deux additifs choisis parmi les additifs organiques ou les charges minérales qui présentent une capacité d'émission et/ou absorption de radiations infrarouges dans la plage de longueur d'onde située entre 2µm et 20µm, pour augmenter l'élasticité de la peau par émission et/ou absorption de radiations infrarouges dans la plage de longueur d'onde située entre 2 µm et 20µm; les charges minérales étant choisies parmi les oxydes, sulfates, carbonates, phosphates et silicates, et présentant une taille moyenne de particule inférieure ou égale à 2µm ; les additifs organiques étant choisis parmi les composés organiques comprenant du silicium et les composés organiques métalliques.

Les polymères utilisés sont ceux filés à l'état fondu tels que le polyester, le polyamide, les polyoléfines (et leurs copolymères), entre autres, ou à travers des solutions, tels que les polymères polyacryliques, les polyacrylates et leurs copolymères, et les dérivés de cellulose tels que l'acétate de cellulose, le propionate de cellulose, la viscose, etc. Les additifs peuvent être introduits dans le polymère selon une méthode quelconque connue de l'homme du métier. Préférentiellement, l'introduction est réalisée dans la phase de synthèse du polymère, ou bien dans la phase de filage au moyen d'un mélange direct des charges minérales et/ou additifs organiques dans le polymère fondu ou en solution, ou encore à travers un mélange maître (« masterbatch »), l'utilisation d'une combinaison de deux modes d'introduction pouvant être appropriée.

La composition selon l'invention comprend une combinaison d'additifs organiques ou charges minérales qui présentent une capacité d'émission et/ou absorption de radiations infrarouges dans la plage de longueur d'onde située entre 2µm et 20µm, et d'un polymère.

La composition selon l'invention présente un nombre de pics d'absorption de radiations infrarouges supérieur à 10 dans les dix plages de fréquence suivantes : 3,00 +/- 0,30µm, 6,20 +/- 0,50µm, 8,00 +/- 0,25µm, 8,50 +/- 0,25µm, 9,00 +/- 0,25µm, 9,50 +/- 0,25µm, 10,00 +/- 0,25µm, 10,50 +/- 0,25µm, 11,00 +/- 0,25µm, 14,60 +/- 2,10µm, au moins 1 pic étant présent dans au moins 7 de ces dix plages de fréquence.

Le spectre d'absorption de radiations infrarouges de la composition peut être déterminé par toute méthode connue de l'homme du métier. Une méthode possible est l'utilisation d'un appareil Bruker Equinox 55, avec une résolution de 4 cm⁻¹. Dans ce cas le spectre obtenu est sous forme ATR (« Atenuated Total Reflectance »), en utilisant un cristal ZnSe.

Avantageusement les charges minérales sont d'au moins un type choisi parmi les oxydes, sulfates, carbonates, phosphates et silicates, présentant une taille moyenne de particule inférieure à 2µm.

Selon la présente invention, on fournit une composition de polymère qui inclut des additifs émetteurs d'infrarouge dans la plage de longueur d'onde située entre 2µm et 20µm. Le polymère peut être choisi dans le groupe comprenant les polyesters, les polyoléfines, les polymères à base de cellulose-ester tels que l'acétate de cellulose, le propionate de cellulose, le rayon, la viscose et les polymères de la même famille, les polymères et copolymères acryliques, les polyamides, le polyhexaméthylène adipamide (PA66) ou le polycaproamide (PA6), ou leurs copolymères en toutes proportions, ou encore des mélanges entre n'importe quels polymères cités précédemment. Selon une forme préférentielle de réalisation, le polymère thermoplastique qui compose la matrice thermoplastique de la composition polymérique est à base de polyamide, choisi entre le polyamide 6, le polyamide 66 et les copolymères de polyamide 6/polyamide 66 en toutes proportions.

Des additifs ont été développés, et qui peuvent être utilisés dans la production par exemple de fils, fibres et filaments ayant des propriétés biostimulantes qui offrent une meilleure élasticité de la peau, résultant en un plus grand confort et bien-être pour les utilisateurs des articles les contenant, en particulier les utilisateurs ayant de la cellulite. Ces articles permettent de diminuer la cellulite.

Plus précisément, la présente invention se rapporte en premier lieu à l'utilisation d'une association d'additifs dans des compositions polymériques pour obtenir l'effet décrit ci-dessus, caractérisé en ce que l'association comprend au moins une charge minérale choisie parmi le groupe des oxydes (dioxyde de titane, dioxyde de silicium, oxyde de magnésium), le groupe des sulfates (sulfate de baryum, sulfate de calcium, sulfate de strontium), le groupe des carbonates (carbonate de calcium ou de sodium), le groupe des silicates (actinolite, tourmaline, serpentine, kaolin et autres aluminosilicates) et le groupe des phosphates (phosphates de zirconium, apatite, ainsi que d'autres possibles, ou encore leurs mélanges).

Les additifs organiques peuvent être des composés organiques contenant du silicium, comme les organoalcoxy silanes, par exemple le diméthyl diéthoxy silane, ou le méthyl triethoxy silane. Les additifs organiques peuvent également être des composés organiques métalliques comme par exemple l'ester alkyle métallique, le chélate d'alkyle métallique, l'acylate d'alkyle métallique, le chélate d'ester métallique, l'alcoolate métallique, l'isopropylate d'aluminium, le sec-butylate d'aluminium ou le tétra acétylacétonate de zirconium, préférentiellement l'isopropylate d'aluminium, le sec-butylate d'aluminium ou le tétra acétylacétonate de zirconium.

Les charges minérales utilisées en association comme absorbeurs et/ou émetteurs d'infrarouge dans la plage de longueur d'onde située entre 2µm et 20µm se présentent sous forme de particules de taille inférieure à 2µm, préférentiellement inférieure à 1µm et avantageusement inférieure à 0,5µm. Les particules peuvent être avantageusement enveloppées ou recouvertes pour les rendre inertes aux composants auxquels elles seront incorporées ou encore pour procurer une meilleure compatibilité avec le substrat polymérique, sans que cela n'intervienne dans leur caractéristique d'absorbeurs et/ou émetteurs d'infrarouge dans la plage considérée.

Les associations de deux charges minérales, ou de trois charges minérales, sont préférées, et en particulier les associations ternaires peuvent être choisies parmi celles qui comprennent le dioxyde de titane, le sulfate de baryum, le dioxyde de silicium et une charge du groupe des silicates.

Encore plus particulièrement, l'association comprend trois charges minérales en mélange de proportions quelconques, telles que celles choisies dans le groupe comprenant : dioxyde de titane/ dioxyde de silicium/ tourmaline ; dioxyde de titane/ dioxyde de silicium/ sulfate de baryum; et dioxyde de titane/ sulfate de baryum/ tourmaline. Préférentiellement on emploie le dioxyde de titane/ sulfate de baryum/ tourmaline.

Selon la présente invention, l'association de charges minérales décrite ci-dessus est utilisée comme additif émetteur d'infrarouge dans la plage de 2µm à 20µm dans des compositions polymériques pour la production de fils, fibres, filaments et articles textiles.

L'additif est présent selon un mode particulier de réalisation de l'invention en quantité inférieure à 9,0% d'additif par rapport à la masse totale de la composition de polymère, de préférence inférieure à 6,0%, avantageusement inférieure à 4,5% en poids. De même, selon un autre mode particulier de réalisation de l'invention, la proportion en poids de l'association de charges minérales par rapport au poids total de la composition polymérique est supérieure à 1,0%, de préférence supérieure ou égale à 1,5% et plus préférentiellement encore supérieure ou égale a 2,5%.

La composition de polymère peut encore contenir un agent antimicrobien ou bactériostatique, ignifugeant, stabilisant face aux rayons UV, ainsi que d'autres agents connus de l'homme du métier.

Selon la présente invention, il est possible d'utiliser une association d'additifs telle que celle décrite ci-dessus en proportions quelconques. À titre d'exemple, et de manière non limitative, les charges minérales des associations ternaires seront réalisées dans l'utilisation de la présente invention en proportions variant avantageusement de 80:10:10 à 10:30:60, plus spécifiquement en proportions de 50:25:25.

Un autre objet de la présente invention est le procédé de préparation des compositions polymériques avec une association de charges minérales absorbeuses/émettrices d'infrarouge distant tel que défini précédemment. Les charges ou additifs peuvent êtres introduits dans la composition polymérique selon une méthode quelconque connue de l'homme du métier. Préférentiellement, l'introduction est réalisée au cours de la phase de synthèse du polymère, ou par mélange directement au polymère au cours de la phase de filage des filaments, ou encore au moyen d'un concentré de particules sous forme de « masterbatch », postérieurement dilué en concentrations prédéterminées dans la masse polymérique au cours de la phase de filage.

Les charges minérales ou additifs organiques peuvent être additionnés séparément selon l'une ou plusieurs méthodes d'introduction décrites ci-dessus.

Le mélange maître est préparé avec des quantités de charge minérale comprises avantageusement entre 10% et 65% en poids par rapport à sa masse totale, de préférence entre 15% et 35%, encore plus préférentiellement entre 15% et 25%.

La présente invention se rapporte également aux articles et en particulier aux fils, fibres et filaments obtenus à partir des compositions décrites ci-dessus, dans lesquelles l'association des charges minérales ou additifs organiques de la présente invention a été utilisée.

Dans le cas de fils, fibres et filaments obtenus par filage à l'état fondu, la composition thermoplastique additivée est obtenue avec l'introduction des charges minérales ou additifs organiques dans le polymère fondu au moyen d'un dispositif de mélange, par exemple en amont d'un dispositif de filage. Par le filage de la composition thermoplastique additivée on peut obtenir des fils multifilamentaires continus, des monofilaments, des fibres courtes et longues, ou leurs mélanges. Les fils, fibres et filaments obtenus à partir des compositions polymériques présentées dans la présente invention peuvent être soumis à tous les traitements textiles connus de l'homme du métier, tels qu'extrusion, étirage, texturation, teinture, finition etc.

La présente invention se rapporte également aux articles obtenus à partir des fils, fibres et filaments décrits ci-dessus. Les articles peuvent être obtenus à partir d'un seul type de fil, fibre ou filament, ou à partir d'un mélange de fils, fibres ou filaments de types différents.

Par articles, on entend notamment les tissus, les tricots et les non-tissés. L'article peut être composé d'au moins un type de fil, filament ou fibre obtenu à partir de compositions polymériques décrites dans la présente invention.

L'article peut également être un film ou une poudre obtenus à partir de la composition décrite ci-dessus. Le film ou la poudre peuvent être obtenus selon toute méthode connue de l'homme du métier.

La présente invention se rapporte aussi à l'utilisation d'un'article, en particulier textile, tel que décrit ci-dessus, à base d'une composition telle que décrite ci-dessus, pour stimuler des tissus biologiques, en particulier des tissus biologiques de sportifs. Avantageusement le tissu biologique est la peau, en particulier la peau de personnes ayant de la cellulite.

Les exemples suivants présentés à titre indicatif feront bien ressortir les avantages de la présente invention.

### EXEMPLES

Les échantillons des exemples 1 et 2 ci-dessous ont été préparés avec un polyamide 66 de viscosité relative (VR) 43, mesurée dans une solution d'acide formique à 90% dans l'eau. L'incorporation de charges minérales émettrices d'infrarouge dans le polyamide 66 a été réalisée à travers le mélange des charges minérales sous forme de poudre et du polymère trituré, dans une proportion de 20% en poids de charge minérale pour l'obtention d'un mélange maître. Le mélange a été extrudé, refroidi et granulé. Le « masterbatch » ainsi obtenu a été introduit dans le polyamide 66 au cours de la phase de filage. La composition polymérique fondue a été filée à une température entre 280°C et 300°C (mesurée dans la filière), refroidie à l'air (20°C, humidité relative de 65%) et enroulée à une vitesse de 4200m/min pour obtenir un fil continu multifilamentaire. Le fil multifilamentaire formé de 68 filaments de section circulaire a été postérieurement texturé. Le titre du filament dans le produit fini est de 1,2dtex. Le fil ainsi obtenu a été utilisé dans la production de tricots pour la confection de bermudas et tee-shirts, par utilisation d'une tricoteuse circulaire. Les tee-shirts ainsi obtenus présentent une densité de surface de 175 g/m², et les bermudas une densité de surface de 305 g/m², et contiennent 12% d'élasthanne. Ces articles ont ensuite été utilisés pour évaluer la performance des compositions.

### Exemples 1 et 2

### Elasticité de la peau

On a mesuré la variation d'élasticité de la peau (sur une région comprenant le côté extérieur, l'avant et l'arrière de la cuisse et de la fesse) d'un groupe de 15 volontaires avant porté pendant 60 jours, 6 heures par jour, un bermuda dont une jambe a été fabriquée à l'aide du fil de l'exemple 1, et l'autre jambe a été fabriquée à l'aide du fil de l'exemple 2. Les 15 volontaires présentent un degré de cellulite I ou II sur l'échelle « NURNBERGER-MULLER SCALE » (Référence : Nurnberger F. Muller G. So-called cellulite: an invented disease. J Dermatol Surg Oncol 1978; 4: 221-229).

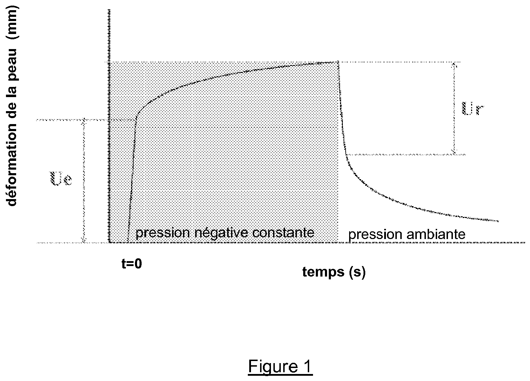

L'élasticité de la peau a été mesurée à l'aide de l'appareil Cutometer ® MPA580 commercialisé par la société CK Electronic GmbH qui utilise le principe de la méthode de succion. Une pression négative est créée dans l'appareil et la peau est tirée dans l'ouverture de la sonde. A l'intérieur de la sonde, on mesure la profondeur de pénétration de la sonde. La capacité de la peau à retourner à sa position initiale lorsque la pression négative n'est plus appliquée (élasticité) est mesurée et exprimée à l'aide d'une courbe (voir figure 1).

L'élasticité de la peau correspond au rapport R=Uᵣ/Uₑ, les valeurs de Ur et Ue correspondant aux valeurs indiquées sur la figure 1.

La variation d'élasticité F correspond à F=[(R_{f}-Ri)/Rᵢ]*100 avec Rᵢ qui correspond à l'élasticité avant les 60 jours, et Rf qui est l'élasticité après les 60 jours.

Le tableau 1 ci-dessous résume la variation d'élasticité F obtenue pour le fil polyamide 66 contenant 1,5% de TiO2 et pour le polyamide 66 contenant 1,5% de TiO2 (exemple 1) et 0,5% de BaSO4 et 0,2% de tourmaline (exemple 2).

**Tableau 1**

| Échantillon | F (%) |
|---|---|
| Polyamide 66 contenant 1,5% de TiO2 | 1 |
| Polyamide 66 TiO2 + BaSO4 + tourmaline | 8 |

Les résultats du tableau ci-dessus montrent une augmentation significative d'élasticité pour le fil polyamide 66 contenant 1,5% de TiO2, 0,5% de BaSO4 et 0,2% de tourmaline que pour le fil polyamide 66 contenant 1,5% de TiO2. Ces résultats montrent l'influence de l'additif favorisant l'augmentation de l'élasticité de la peau et donc la diminution de la cellulite.

L'augmentation de l'élasticité de la peau est associées à un meilleur confort et bien-être, et à une diminution de la cellulite. Ainsi, les résultats présentés dans les exemples ci-dessus indiquent un meilleur confort et bien-être en rapport avec l'usage de l'article produit avec les fils de polyamide additivé par l'emploi de charges minérales ou additifs organiques émetteurs d'infrarouge distant définis dans la présente invention.

### Exemple 3

L'échantillon ci-dessous a été préparés avec un polyamide 66 de viscosité relative (VR) 43, mesurée dans une solution d'acide formique à 90% dans l'eau. L'incorporation du TiO2 et de la tourmaline dans le polyamide 66 est réalisée par introduction de ces charges lors du procédé de polymérisation du polyamide 66, sous la forme d'une suspension aqueuse de TiO2 à 20%, et d'une suspension aqueuse de tourmaline à 39%. L'incorporation du BaSo4 dans le polyamide 66 a été réalisée à travers le mélange des charges minérales sous forme de poudre et du polyamide 66, dans une proportion de 20% en poids de BaSO4 pour l'obtention d'un mélange maître. Le mélange a été extrudé, refroidi et granulé. Le « masterbatch » ainsi obtenu a été introduit dans le polyamide 66 au cours de la phase de filage. La composition polymérique fondue a été filée à une température entre 280°C et 300°C (mesurée dans la filière), refroidie à l'air (20°C, humidité relative de 65%) et enroulée à une vitesse de 4200m/min pour obtenir un fil continu multifilamentaire. Le fil multifilamentaire formé de 68 filaments de section circulaire a été postérieurement texturé. Le titre du filament dans le produit fini est de 1,2dtex. Le fil ainsi obtenu a été utilisé dans la production de tricots pour la confection de bermudas, par utilisation d'une tricoteuse circulaire. Les bermudas ainsi obtenus présentent une densité de surface de 305 g/m², et contiennent 12% d'élasthanne. Ces articles ont ensuite été utilisés pour évaluer la performance des compositions.

Un échantillon de fil de polyamide 66 contenant 1,5% de TiO2, 0,5% de BaSO4 et 0,2% de tourmaline a été préparé selon la description précédente.

Cette composition présente les propriétés d'absorption de radiations infrarouges suivantes :
- nombre de pics dans la plage de fréquence 3,00 +/- 0,30µm : 2
- nombre de pics dans la plage de fréquence 6,20 +/- 0,50µm : 2
- nombre de pics dans la plage de fréquence 8,00 +/- 0,25µm : 1
- nombre de pics dans la plage de fréquence 8,50 +/- 0,25µm : 1
- nombre de pics dans la plage de fréquence 9,00 +/- 0,25µm : 0
- nombre de pics dans la plage de fréquence 9,50 +/- 0,25µm : 1
- nombre de pics dans la plage de fréquence 10,00 +/- 0,25µm : 0
- nombre de pics dans la plage de fréquence 10,50 +/- 0,25µm : 2
- nombre de pics dans la plage de fréquence 11,00 +/- 0,25µm : 0
- nombre de pics dans la plage de fréquence 14,60 +/- 2,10µm : 3

## Revendications

1. Utilisation non thérapeutique d'un article à base d'une composition polymérique comprenant un polymère et au moins deux additifs choisis parmi les additifs organiques ou les charges minérales qui présentent une capacité d'émission et/ou absorption de radiations infrarouges dans la plage de longueur d'onde située entre 2µm et 20µm, pour augmenter l'élasticité de la peau par émission et/ou absorption de radiations infrarouges dans la plage de longueur d'onde située entre 2 µm et 20µm; les charges minérales étant choisies parmi les oxydes, sulfates, carbonates, phosphates et silicates, et présentant une taille moyenne de particule inférieure ou égale à 2µm ; les additifs organiques étant choisis parmi les composés organiques comprenant du silicium et les composés organiques métalliques.

2. Utilisation selon la revendication 1, **caractérisée en ce que** le polymère est choisi parmi les polyesters, les polyoléfines, les polymères cellulosiques tels que l'acétate de cellulose, le propionate de cellulose, le rayon, la viscose, les polymères et copolymères acryliques, et les polyamides en général, comme le polyamide 66 ou le polyamide 6, ou leurs copolymères ou leurs mélanges.

3. Utilisation selon la revendication 2, **caractérisée en ce que** le polymère est à base de polyamide.

4. Utilisation selon la revendication 3, **caractérisée en ce que** le polymère qui compose la matrice de la composition polymérique est un polyamide choisi entre le polyamide 6, le polyamide 66 et les copolymères de polyamide 6/polyamide 66.

5. Utilisation selon l'une des revendications précédentes, **caractérisée en ce que** l'oxyde est choisi parmi le dioxyde de titane, le dioxyde de silicium et l'oxyde de magnésium.

6. Utilisation selon l'une des revendications précédentes, **caractérisée en ce que** le sulfate est choisi parmi le sulfate de baryum, le sulfate de calcium et le sulfate de strontium.

7. Utilisation selon l'une des revendications précédentes, **caractérisée en ce que** le carbonate est choisi parmi le carbonate de calcium ou de sodium.

8. Utilisation selon l'une des revendications précédentes, **caractérisée en ce que** le silicate est choisi parmi l'actinolite, la tourmaline, la serpentine, le kaolin et d'autres aluminosilicates.

9. Utilisation selon l'une des revendications précédentes, **caractérisée en ce que** le phosphate est choisi parmi les phosphates de zirconium, l'apatite ou leurs mélanges.

10. Utilisation selon l'une des revendications précédentes, **caractérisée en ce que** les charges minérales sont choisies parmi le dioxyde de titane, le sulfate de baryum, et une charge du groupe des silicates.

11. Utilisation selon l'une des revendications précédentes, **caractérisée en ce que** la composition polymérique comprend deux charges minérales.

12. Utilisation selon l'une des revendications précédentes, **caractérisée en ce que** la composition polymérique comprend trois charges minérales.

13. Utilisation selon la revendication 12, **caractérisée en ce que** la proportion en poids des trois charges est comprise entre 80:10:10 et 10:30:60.

14. Utilisation selon la revendication 12 ou 13, **caractérisée en ce que** l'association des trois charges minérales est l'association dioxyde de titane/ sulfate de baryum/ tourmaline

15. Utilisation selon l'une des revendications précédentes, **caractérisée en ce que** la proportion en poids de l'association de charges minérales par rapport au poids total de la composition polymérique est supérieure à 1,0%, de préférence supérieure ou égale à 1,5% et plus préférentiellement encore supérieure ou égale a 2,5%.

16. Utilisation selon l'une des revendications précédentes, **caractérisée en ce que** la proportion en poids de l'association de charges minérales par rapport au poids total de la composition polymérique est inférieure à 9%, de préférence inférieure à 6%, avantageusement inférieure à 4,5%.

17. Utilisation selon l'une des revendications précédentes, **caractérisée en ce que** l'article se présente sous la forme de fils, fibres, filaments, ou un mélange de ceux-ci, tissus, non tissé ou tricot, ou d'un film ou d'une poudre.

## Patentansprüche

1. Nichttherapeutische Verwendung eines Artikels auf Basis einer Polymerzusammensetzung, umfassend ein Polymer und mindestens zwei Additive, die aus organischen Additiven oder mineralischen Füllstoffen, die ein Emissions- und/oder Absorptionsvermögen für Infrarotstrahlung im Wellenlängenbereich zwischen 2 µm und 20 µm aufweisen, ausgewählt sind zur Erhöhung der Elastizität der Haut durch Emission und/oder Absorption von Infrarotstrahlung im Wellenlängenbereich zwischen 2 µm und 20 µm; wobei die mineralischen Füllstoffe aus Oxiden, Sulfaten, Carbonaten, Phosphaten und Silikaten ausgewählt sind und eine mittlere Teilchengröße von weniger als oder gleich 2 µm aufweisen; wobei die organischen Additive aus organischen Verbindungen, die Silicium enthalten, und metallorganischen Verbindungen ausgewählt sind.

2. Verwendung nach Anspruch 1, **dadurch gekennzeichnet, dass** das Polymer aus Polyestern, Polyolefinen, Cellulosepolymeren, wie Celluloseacetat, Cellulosepropionat, Reyon und Viskose, Acrylpolymeren und -copolymeren und Polyamiden im Allgemeninen, wie Polyamid 66 oder Polyamid 6, oder deren Copolymereb oder deren Gemischen ausgewählt ist.

3. Verwendung nach Anspruch 2, **dadurch gekennzeichnet, dass** das Polymer auf Polyamid basiert.

4. Verwendung nach Anspruch 3, **dadurch gekennzeichnet, dass** es sich bei dem Polymer, das die Matrix der Polymerzusammensetzung ausmacht, um ein Polyamid handelt, das aus Polyamid 6, Polyamid 66 und den Copolymeren von Polyamid 6/Polyamid 66 ausgewählt ist.

5. Verwendung nach einem der vorhergehenden Ansprüche, **dadurch gekennzeichnet, dass** das Oxid aus Titandioxid, Siliciumdioxid und Magnesiumoxid ausgewählt ist.

6. Verwendung nach einem der vorhergehenden Ansprüche, **dadurch gekennzeichnet, dass** das Sulfat aus Bariumsulfat, Calciumsulfat und Strontiumsulfat ausgewählt ist.

7. Verwendung nach einem der vorhergehenden Ansprüche, **dadurch gekennzeichnet, dass** das Carbonat aus Calcium- oder Natriumcarbonat ausgewählt ist.

8. Verwendung nach einem der vorhergehenden Ansprüche, **dadurch gekennzeichnet, dass** das Silikat aus Actinolit, Turmalin, Serpentin, Kaolin und anderen Aluminosilikaten ausgewählt ist.

9. Verwendung nach einem der vorhergehenden Ansprüche, **dadurch gekennzeichnet, dass** das Phosphat aus Zirconiumphosphaten und Apatit oder deren Mischungen ausgewählt ist.

10. Verwendung nach einem der vorhergehenden Ansprüche, **dadurch gekennzeichnet, dass** die mineralischen Füllstoffe aus Titandioxid, Bariumsulfat und einem Füllstoff aus der Gruppe der Silikate ausgewählt ist.

11. Verwendung nach einem der vorhergehenden Ansprüche, **dadurch gekennzeichnet, dass** die Polymerzusammensetzung zwei mineralische Füllstoffe umfasst.

12. Verwendung nach einem der vorhergehenden Ansprüche, **dadurch gekennzeichnet, dass** die Polymerzusammensetzung drei mineralische Füllstoffe umfasst.

13. Verwendung nach Anspruch 12, **dadurch gekennzeichnet, dass** der Gewichtsanteil der drei Füllstoffe zwischen 80:10:10 und 10:30:60 liegt.

14. Verwendung nach Anspruch 12 oder 13, **dadurch gekennzeichnet, dass** es sich bei der Kombination der drei Füllstoffe um die Kombination Titandioxid/Bariumsulfat/Turmalin handelt.

15. Verwendung nach einem der vorhergehenden Ansprüche, **dadurch gekennzeichnet, dass** der Gewichtsanteil der Kombination von mineralischen Füllstoffen, bezogen auf das Gesamtgewicht der Polymerzusammensetzung, größer als 1,0%, vorzugsweise größer gleich 1,5% und noch weiter bevorzugt größer gleich 2,5% ist.

16. Verwendung nach einem der vorhergehenden Ansprüche, **dadurch gekennzeichnet, dass** der Gewichtsanteil der Kombination von mineralischen Füllstoffen, bezogen auf das Gesamtgewicht der Polymerzusammensetzung, weniger als 9%, vorzugsweise weniger als 6%, vorteilhafterweise weniger als 4,5%, beträgt.

17. Verwendung nach einem der vorhergehenden Ansprüche, **dadurch gekennzeichnet, dass** der Artikel in Form von Fäden, Fasern, Filamenten oder einem Gemisch davon, Geweben, Vliesstoffen oder Maschenwaren, oder eines Films oder eines Pulvers vorliegt.

## Claims

1. Non therapeutic use of an article based on a polymeric composition comprising a polymer and at least two additives chosen from organic additives or inorganic fillers which have a capacity for emission and/or absorption of infrared radiation in the wavelength range of between 2 µm and 20 µm, for increasing the elasticity of the skin by emission and/or absorption of infrared radiation in the wavelength range of between 2 µm and 20 µm; the inorganic fillers being chosen from oxides, sulphates, carbonates, phosphates and silicates, and having an average particle size of less than or equal to 2 µm; the organic additives being chosen from organic compounds comprising silicon and metallic organic compounds.

2. Use according to Claim 1, **characterized in that** the polymer is chosen from polyesters, polyolefins, cellulose-based polymers such as cellulose acetate, cellulose propionate, rayon and viscose, acrylic polymers and copolymers, and polyamides in general, such as polyamide 66 or polyamide 6, or copolymers thereof or blends thereof.

3. Use according to Claim 2, **characterized in that** the polymer is based on polyamide.

4. Use according to Claim 3, **characterized in that** the polymer of which the matrix of the polymeric composition is composed is a polyamide chosen from polyamide 6, polyamide 66 and polyamide 6/ polyamide 66 copolymers.

5. Use according to one of the preceding claims, **characterized in that** the oxide is chosen from titanium dioxide, silicon dioxide and magnesium oxide.

6. Use according to one of the preceding claims, **characterized in that** the sulphate is chosen from barium sulphate, calcium sulphate and strontium sulphate.

7. Use according to one of the preceding claims, **characterized in that** the carbonate is chosen from calcium carbonate or sodium carbonate.

8. Use according to one of the preceding claims, **characterized in that** the silicate is chosen from actinolite, tourmaline, serpentine, kaolin and other aluminosilicates.

9. Use according to one of the preceding claims, **characterized in that** the phosphate is chosen from zirconium phosphates and apatite, or mixtures thereof.

10. Use according to one of the preceding claims, **characterized in that** the inorganic fillers are chosen from titanium dioxide, barium sulphate and a filler of the silicate group.

11. Use according to one of the preceding claims, **characterized in that** the polymeric composition comprises two inorganic fillers.

12. Use according to one of the preceding claims, **characterized in that** the polymeric composition comprises three inorganic fillers.

13. Use according to Claim 12, **characterized in that** the proportion by weight of the three fillers is between 80:10:10 and 10:30:60.

14. Use according to Claim 12 or 13, **characterized in that** the combination of the three inorganic fillers is the titanium dioxide/barium sulphate/ tourmaline combination.

15. Use according to one of the preceding claims, **characterized in that** the proportion by weight of the combination of inorganic fillers relative to the total weight of the polymeric composition is greater than 1.0%, preferably greater than or equal to 1.5% and even more preferentially greater than or equal to 2.5%.

16. Use according to one of the preceding claims, **characterized in that** the proportion by weight of the combination of inorganic fillers relative to the total weight of the polymeric composition is less than 9%, preferably less than 6%, advantageously less than 4.5%.

17. Use according to one of the preceding claims, **characterized in that** the article is in the form of yarns, fibres, filaments, or a mixture thereof, fabrics, a nonwoven or a knit, or of a film or of a powder.
